# EUROPEAN PATENT APPLICATION

(11) **EP 4 362 042 A1**
(43) Date of publication of application: **01.05.2024**
(21) Application number: 22204671.6
(22) Date of filing: 31.10.2022
(51) Int. Cl.: G16H 30/20, G16H 30/40, G16H 50/20

(54) **ESTIMATING PHARMACOKINETICS OF A CONTRAST MEDIUM THROUGH A CARDIOVASCULAR SYSTEM ANALYTICAL MODEL AND/OR NEURAL NETWORK ALGORITHM**

(71) Applicant: Siemens Healthineers AG, 91301 Forchheim (DE); Duke University, Durham, North Carolina 27705 (US)
(72) Inventor:
(74) Representative: Siemens Healthineers Patent Attorneys

(57) **Abstract**

According to various examples, pharmacokinetics of a contrast medium through a cardiovascular system of a patient or estimated. This is achieved using analytical model such as a compartment model (2051) or a linear time invariant model (2052), and/or one or more neural network algorithms (2010).

## Description

Various examples of the disclosure pertain to techniques of estimating hemodynamic properties of a patient. Various examples of the disclosure specifically pertain to estimating pharmacokinetics of a contrast medium through the cardiovascular system of a patient. Various examples pertain to configuring an angiographic imaging protocol based on such information.

Contrast-medium enhanced angiographic imaging protocols are used to investigate the cardiovascular system of a patient, e.g., the vessel lumen, stenosis, thrombosis, calcified plaque, organ perfusion, arterial input function, etc. Angiographic imaging protocols may be used in the field of oncology.

For instance, medical interventions in vessels to treat pathological vessel conditions, such as obstructions, typically rely on a catheter or some other means. Depending on the specific pathological vessel to be treated, the catheter is inserted into the vessels, e.g., starting at an antecubital vein or in the femoral vein and advanced via major vessels to reach the general vicinity of the pathological vessel. Since vessels tend to branch extensively, at least starting in the general vicinity of the pathological vessel a medical specialist or operator relies on imaging methods based on contrast media and radiation, such as fluoroscopy, to determine the path through the vessel to reach the pathological vessel. Depending on the position of the pathological vessel - defining the region of interest (ROI) - in the vessel tree, the injection of the contrast medium needs to be adjusted. Also, image acquisition is to be synchronized with arrival of the contrast medium at the ROI.

Accordingly, the quality and diagnostic value of the angiographic imaging data relies on the imaging system (e.g., capabilities and/or configuration), the training/experience of the staff, but also on injection- and patient-specific factors.

Various analytical models to estimate the pharmacokinetics of a contrast medium through the cardiovascular system are known. One example is a so-called Compartment Model (CoM) that relies on multiple compartments associated with vessels and organs and that models the spatio-temporal concentration of the contrast medium by differential equations. The differential equations model the compartment-specific concentration change rate as a function of the local concentration, as well as inflow and outflow. Examples are described, e.g., in Sahbaee, Pooyan, et al. "The effect of contrast material on radiation dose at CT: Part I. Incorporation of contrast material dynamics in anthropomorphic phantoms." Radiology 283.3 (2017): 739.

Another example is the Linear Time Invariant Model (LTIM). Here, for a given position in the aorta of the patient, the outflow concentration over time can be determined as a convolution of the patient-specific response function and the in-flow concentration over time . Details are described in US 9,271,656 B2.

However, while such analytical models are comprehensive and show acceptable results for timing adaption, they oftentimes lack precision and accuracy in terms of contrast enhancement prediction, especially in presence of contrast dynamic influencing pathologies (stenosis, bleedings, dissections, tumor related angiogenesis, fibrosis, cirrhosis, etc.).

To apply methods based on artificial intelligence (specifically neural network algorithms, NNs) is another technique proposed before, as they can learn a contrast-medium enhancement curve in an organ more precisely. See, e.g., US 10,925,565 B2. However, NNs oftentimes lack interpretability, need significantly more training data than analytical models, and are also prone to adversarial attacks, i.e., a small deviation in the data used during inference from training data might lead to completely implausible predictions.

### SUMMARY

Accordingly, there is a need for advanced techniques of configuring an angiographic imaging protocol. A need exists for techniques that mitigate or overcome at least some of the above-identified restrictions and drawbacks.

This need is met by the features of the independent claims. The features of the dependent claims define embodiments.

Hereinafter, techniques of configuring an angiographic imaging protocol are disclosed. The angiographic imaging protocol is configured based on one or more estimates of pharmacokinetics of a contrast medium through a cardiovascular system of a patient. Various techniques are disclosed that facilitate determining the one or more estimates of the pharmacokinetics. Analytical models and/or neural network algorithms or, more generally, machine learning can be employed to determine the estimates. Techniques are disclosed that enable to rely on a combination of analytical models and neural network algorithms to achieve the task, thereby obtaining more accurate results.

In an example, a computer-implemented method includes performing a training of a neural network algorithm. The neural network algorithm is trained to estimate pharmacokinetics of a contrast medium through a cardiovascular system of a patient. The training is based on a measurement indicative of a reference contrast medium bolus. The method also includes configuring an angiographic imaging protocol based on an estimate of the pharmacokinetics of the contrast medium. The estimate is obtained from the neural network algorithm. The at least one algorithm includes a loss that is determined based on a compartment model of the cardiovascular system. The compartment model is based on a fragmentation of the cardiovascular system into multiple compartments, as well as associated differential equations that define a change of a concentration of the contrast medium as a function of time and the concentration of the contrast medium at the respective compartment.

Accordingly, the method can include the training of the neural network algorithm as well as inference to obtain an estimate of the pharmacokinetics without available ground truth.

The neural network algorithm can accordingly obtain, as input, at least a measurement that is indicative of the reference contrast medium bolus. This can include bolus tracking or a test bolus measurement.

The use of the at least one loss that is determined based on the compartment model is only one example. Other examples are possible.

In an example, a computer-implemented method includes performing a training of a neural network algorithm. The neural network algorithm is trained to estimate pharmacokinetics of a contrast medium through a cardiovascular system of a patient based on a measurement that is indicative of a reference contrast medium bolus. The training is dependent on at least one loss. The method also includes configuring an angiographic imaging protocol based on an estimate of the pharmacokinetics of the contrast medium that is obtained from the neural network algorithm. The at least one loss includes a loss that is determined based on a linear time invariant model of the cardiovascular system. The linear time invariant model is based on a response function of a vessel of the cardiovascular system with respect to a time-dependent inflow of the contrast medium.

The use of the reference contrast medium bolus measurement as input to the neural network algorithm is an example. In further examples, further types of input data can be considered additionally or alternatively.

In an example, a computer-implemented method includes obtaining an estimate of pharmacokinetics of a contrast medium through a cardiovascular system of a patient. The estimate is obtained from a neural network algorithm. The neural network algorithm in turn obtains, as input, at least a three-dimensional image of the patient. The computer-implemented method also includes configuring an angiographic imaging protocol based on the estimate of the pharmacokinetics of the contrast medium.

In various examples, a neural network algorithm can be used to determine the estimate of the pharmacokinetics of the contrast medium. In some examples, the neural network algorithm can be complemented or replaced by an analytical model.

In an example, a computer-implemented method includes determining one or more parameters values of at least one free parameter of an analytical model that estimates pharmacokinetics of a contrast medium through a cardiovascular system of a patient. Said determining his implemented using an optimization algorithm. The optimization algorithm operates based on an input that includes a measurement that is indicative of a reference contrast medium bolus. The method also includes configuring the angiographic imaging protocol based on an estimate of the pharmacokinetics of the contrast medium that is obtained from the analytical model.

Thus, the analytical model is initially parametrized using the optimization algorithm; then, the estimate obtained from the parametrized analytical model is used to configure the angiographic imaging protocol.

In an example, a computer-implemented method includes obtaining an estimate of pharmacokinetics of a contrast medium through a cardiovascular system of a patient. The estimate is obtained from a neural network algorithm. The neural network algorithm includes a known operator layer. The known operator layer implements an analytical model. The analytical model also estimates the pharmacokinetics of the contrast medium through the cardiovascular system of the patient. The method also includes configuring an angiographic imaging protocol based on the estimate of the pharmacokinetics of the contrast medium that is obtained from the neural network algorithm.

Accordingly, it is possible to employ multiple models and/or neural network algorithms to estimate the pharmacokinetics of the contrast medium through the cardiovascular system of the patient. According to examples, such estimation techniques can be combined with each other.

In an example, a computer-implemented method includes determining multiple pre-estimates of pharmacokinetics of a contrast medium through a cardiovascular system of the patient. The multiple pre-estimates are determined based on multiple models, e.g., analytical models, and/or neural network algorithms. The method also includes determining an estimate of the pharmacokinetics based on a weighted combination of the multiple pre-estimates. The method further includes configuring and angiographic imaging protocol based on the estimate of the pharmacokinetics.

In an example, a computing device includes at least one processor and a memory. The at least one processor is configured to load program code from the memory and to execute the program code. The at least one processor, upon executing the program code, is configured to perform one or more of various disclosed methods.

A computer program or a computer-program product or a computer-readable storage medium includes program code. The program code can be loaded and executed by at least one processor. The at least one processor, upon executing the program code, is configured to perform one or more of the various disclosed methods.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

FIG. 1 schematically illustrates a Computed Tomography scanner that can be used to execute an angiographic imaging protocol according to various examples.
FIG. 2 schematically illustrates a contrast-medium enhancement curve according to various examples.
FIG. 3 is a flowchart of a method according to various examples.
FIG. 4 schematically illustrates a data-processing pipeline according to various examples.
FIG. 5 schematically illustrates a data-processing pipeline according to various examples.
FIG. 6 schematically illustrates a data-processing pipeline according to various examples.
FIG. 7 is a clinical workflow according to various examples.

### DETAILED DESCRIPTION

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Various techniques relate to configuration of a contrast-medium assisted angiographic imaging protocol. For instance, X-ray, Computed Tomography (CT), or Magnetic Resonance Imaging (MRI) angiographic imaging protocols can be configured using the techniques disclosed herein. Another example would be ultrasound-based angiographic imaging protocol. A typical area of application for contrast medium-assisted imaging is, for example, CT angiography (CTA), that is, the imaging of the vessels by way of computed tomography. A CTA scan is an operation of a computed tomography scanner to image the vessels, and a heart CTA or heart scan is the imaging of the heart and representation of the heart vessels.

Various techniques are based on the finding that administration of a contrast medium typically only provides a comparatively short temporal window for optimally imaging the vasculature of the cardiovascular system, lesions, and/or tumors (for oncology). Thus, optimization of the configuration of the angiographic imaging protocol for accurate synchronization is desirable, both to reduce the dose of the required contrast medium, as well as reproduce the imaging duration. This enables to scan a ROI when the intravascular concentration of the contrast medium is at its peak or, more generally, at a desirable level. For instance, scanning the ROI too early or too late results in suboptimal contrast enhancement and reduced contrast-to-noise ratio. This is because the estimation of stenosis depends on the differentiability between the stenosis itself and the contrast medium. Stenosis are overestimated in case of present blooming artifacts and erroneous spectral imaging, when applied.

Hereinafter, techniques are disclosed that facilitate estimation of one or more hemodynamic properties of a cardiovascular system of an individual patient. Based on such one or more hemodynamic properties, the angiographic imaging protocol can be configured. The angiographic protocol can be configured so that the concentration of the contrast medium is at a desired level, e.g., at its peak. The scan timing can be adjusted.

In detail, one specific hemodynamic property that can be estimated is the pharmacokinetics of the contrast medium through the cardiovascular system of the patient. Examples include, e.g., a volume transfer constant, a fractional volume of the extravascular extracellular space or the volume of the extravascular extracellular space per unit volume of the tissue. Specifically, a contrast enhancement curve for the angiographic imaging protocol due to the contrast medium (contrast-medium enhancement curve), i.e., a time-resolved prediction of the propagation of the contrast medium can be made. Sometimes, the contrast-medium enhancement curve can also be resolved for multiple positions along the cardiovascular system, e.g., different organs and/or different vessels. Then, the angiographic imaging protocol can be configured based on the pharmacokinetics, e.g., the contrast-medium enhancement curve. This helps to synchronize the image acquisition of the angiographic imaging protocol with, e.g., the desired concentration of the contrast medium at a measurement volume or a region of interest of the angiographic imaging protocol. In detail, the desired concentration of the contrast medium is of particular importance in spectral imaging. On the other hand, in non-spectral imaging, the contrast-to-noise ratio (CNR) is a particular decisive value. It depends on the local concentration of the contrast medium, but also local enhancement, which in turn is determined by concentration, applied spectrum and beam hardening effects.

As a general rule, the pharmacokinetics may be estimated on a whole-body level or specifically for an organ or a set of organs and interconnecting vessels. The estimation region may depend on the requirements of the angiographic imaging protocol, e.g., a measurement volume of the angiographic imaging protocol and an administration site at which the contrast medium is administered to the cardiovascular system. To give an example, as a general rule, it would be possible to determine the contrast-medium enhancement curve for a certain position in the cardiovascular system, e.g., for a certain position in the aorta. In another example, it would also be possible to determine a spatial temporal contrast-medium enhancement curve, e.g., for multiple positions throughout the cardiovascular system. For instance, the image-contrast enhancement could be determined for multiple organs or blood vessels. Often, determining contrast-medium enhancement curve for the aorta to make accurate predictions regarding a measurement time duration of the angiographic imaging protocol for various organs.

According to the techniques disclosed herein, it is possible to perform a patient-specific estimation of the pharmacokinetics. This means that a model and/or an algorithm used to estimate the one or more hemodynamic properties can operate based on patient-specific data and/or be parametrized based on patient-specific data. Specifically, it is possible to use measurements that include time series data that is indicative of a reference contrast medium bolus. The reference contrast medium bolus can be applied to the patient or a reference patient.

The reference contrast medium bolus can pertain to a test bolus measurement or a bolus tracking measurement . The test bolus measurement corresponds to a comparatively small (i.e., non-diagnostic) injection of the contrast medium, multiple monitoring scans, and a retrospective analysis in any desired location of an acquired slice. The bolus tracking measurement corresponds to a comparatively large amount of contrast medium being injected (i.e., a diagnostic amount), multiple monitoring scans, and a start delay that defines a time offset between administration of the diagnostic bolus and the measurement.

Alternatively or additionally, patient-specific data, e.g., patient demographics such as age, gender, prior/current clinical conditions (e.g., heart status), cardiac output, injection site, camera images of the patient such as three-dimensional camera data, topograms, specific cardiographic examinations, etc. can be considered to parametrize the model and/or the algorithm, and/or provided as an input to the model and/or the algorithm. By performing a patient-specific estimation of the pharmacokinetics of the cardiovascular system of the patient, a more accurate synchronization of the image acquisition of the angiographic imaging protocol with the desired concentration of the contrast medium at a measurement volume or a region of interest can be achieved.

According to examples disclosed herein, it is possible to determine one or more estimates of pharmacokinetics of a contrast medium through a cardiovascular system of a patient based on one or more analytical models and one or more NNs. This means that it is possible to combine analytical model(s) with a NN on NNs.

According to examples, it is thus possible to collate the analytical and the NN domain. According to examples, it is possible to efficiently combine analytical models based on blood flow dynamics (e.g., a CoM) or systems theory (e.g., an LTIM) with NNs to predict pharmacokinetics of the contrast medium.

Options for analytical models are summarized below in TAB. 1.

**TAB. 1: Various options for analytical models that can be used according to the techniques disclosed herein to estimate the pharmacokinetics of a contrast medium through the cardiovascular system of the patient. In some scenarios, it would also be possible that combinations of the analytical models are relied upon. The analytical models of TAB. 1 are only examples, and further analytical models are based, e.g., models that use fluid-dynamic simulations based on Navier-Stokes equation.**

| MODEL | EXAMPLE DETAILS |
|---|---|
| COMPARTMENT MODEL | The CoM can include a network of multiple compartments that represent key organs and vessels. The CoM can thus be based on a fragmentation of the cardiovascular system into the multiple compartments. |
| | The compartment model can estimate pharmacokinetics of a patient, e.g., a contrast-medium enhancement curve, based on a reference contrast-medium bolus measurement. |
| | Each organ compartment may be further modelled into sub-compartments such as intravascular, extracellular and intracellular compartments. Example compartments are listed in Tab. 1 and FIG. 1 of Sahbaee, Pooyan , et al. "The effect of contrast material on radiation dose at CT: Part I. Incorporation of contrast material dynamics in anthropomorphic phantoms." Radiology 283.3 (2017): 739. |
| | Differential equations can define a change of a concentration of the contrast medium as a function of time and the concentration of the of the contrast medium at the respective compartment. |
| | Ordinary differential equations can describe the change rate of the concentration at each compartment or sub-compartment. This is described in further detail in Bae, Kyongtae T., Jay P. Heiken, and James A. Brink. "Aortic and hepatic contrast medium enhancement at CT. Part I. Prediction with a computer model." Radiology 207.3 (1998): 647-655. |
| | The CoM can thus predict a spatiotemporally resolved contrast-medium enhancement curve. |
| LINEAR TIME INVARIANT MODEL | As a general rule, the LTIM can calculate, for a certain position in the aorta, the respective outflow concentration as a function of the inflow concentration. Accordingly, it is possible to consider a response function of a vessel of the cardiovascular system with respect to a time-dependent inflow of the contrast medium. The response function can be obtained, e.g., from a test bolus measurement. |
| | The LTIM can estimate pharmacokinetics of a patient, e.g., a contrast-medium enhancement curve, based on a reference contrast-medium bolus measurement, e.g., a test bolus measurement or a spatially-resolved test bolus tracking measurement. |
| | The LTIM - different than the CoM - thus does not provide spatially resolved pharmacokinetics. |

Various options are available to collate the analytical and the NN domain. Some options are summarized below in TAB. 2.

**TAB. 2: Various options to use at least two of one or more analytical models and one or more NNs. This corresponds to collating the analytical and the NN domain. The options of TAB. 2 can be combined with each other. For instance, a NN as in option II or III can be based on the NN trained using a physics-informed loss as in option I. is also possible to use a PINN to learn free parameters of an analytical model without a data-driven component in option I as shown by M. Raissi, P. Perdikaris, and G. E. Karniadakis, "Physics-informed NNs: A deep learning framework for solving forward and inverse problems involving nonlinear partial differential equations," Journal of Computational Physics, vol. 378, pp. 686-707, 2019, in the section about "Data-driven discovery of partial differential equations".**

| | BRIEF DESCRIPTION | EXAMPLE DETAILS |
|---|---|---|
| I | Physics-informed loss | The core idea of this approach is to use deviations of the NN estimation from an estimation made by analytical models as additional losses within the training of a NN. This may be termed "physics-informed loss", because the estimation of the analytical model is considered in training the NN. |
| | | According to examples, a training of a NN is performed. The NN is trained to estimate pharmacokinetics of a contrast medium through a cardiovascular system of a patient. The NN is trained to make this estimation based on a measurement indicative of a reference contrast medium bolus, e.g., a bolus tracking measurement or a test bolus measurement. The training depends on at least one loss. The at least one loss can, in particular, include a physics-informed loss that is determined based on at least one analytical model. |
| | | Analytical models as outlined in TAB. 1 may be used, e.g., a CoM or a LTIM. |
| | | For instance, for the CoM, it would be possible that the physics-informed loss is based on a comparison between time and spatial derivatives of a prediction of the NN for the pharmacokinetics of the contrast medium on the one hand, and, on the other hand, further time and spatial derivatives of the concentration of the contrast medium as defined by the differential equations of the CoM. Typically, such an approach is referred to as a physics-informed NN (PINN), see Raissi, Maziar, Paris Perdikaris, and George E. Karniadakis. "Physics-informed NNs: A deep learning framework for solving forward and inverse problems involving nonlinear partial differential equations." Journal of Computational physics 378 (2019): 686-707. Such an approach can be computationally efficient, because respective derivatives of the prediction of the NN are anyway available due to the training being implemented by a gradient descent optimization, referred to as "back propagation". Furthermore, for the CoM, such derivatives are also available as part of the solving of the differential equations using numerical techniques. |
| | | For the LTIM, a distance (e.g., a Euclidian distance or another metric) between the prediction of the NN in its current training state for the pharmacokinetics of the contrast medium (e.g., the contrast medium concentration at a certain position in the order) and the respective prediction of the LTIM can be considered. |
| | | Various types of NNs may be used. For instance, a convolutional NN may be used. It would be possible that the NN includes a neural ordinary differential equation (ODE) algorithm. Those models have its major strength in being compatible with irregularly sampled time series data, which is the case for the task of contrast-medium enhancement curve prediction. |
| | | As a general rule, multiple losses can be used in combination. For instance, a first loss can be based on the Euclidean distance between a measured concentration of the contrast medium (obtained from a reference contrast medium bolus) as a function of time at a specific site in the cardiovascular system, e.g., the aorta, and the estimation of the NN in its current training state; a second (physics-informed) loss can be based on the Euclidean distance to the estimation of the LTIM; and a third (physics-informed) loss could be based on differences between derivatives of the prediction of the NN and derivatives of the concentration of the contrast medium, obtained from the CoM. Where multiple losses are considered, it would be possible to determine weights for the multiple losses, e.g., based on one or more patient-specific parameters such as age, gender, etc. Embedded learning is possible. This helps to more combine the losses in a manner to more accurately train the NN. |
| II | Integrating an optimization algorithm into an analytical model | According to various examples, an analytical model - cf. TAB. 1 - can be used to estimate the pharmacokinetics of the contrast medium through the cardiovascular system of the patient. According to examples, this analytical model can include at least one free parameter. It is then possible to determine the at least one free parameter using an optimization algorithm. |
| | | The optimization algorithm may be selected from the group comprising: a NN; a gradient descent optimization; an iterative numerical optimization that minimizes or maximizes a goal function; a genetic optimization algorithm; a Bayesian optimization algorithm. |
| | | The optimization algorithm can operate based on an input that includes a measurement for the patient that is indicative of a reference contrast medium bolus. Thereby, a patient-specific parameterization of the analytical model can be obtained. |
| | | The at least one free parameter may include, e.g., a time-dependent inflow of concentration of the contrast-medium measurement bolus. Thus, the injection sub-protocol of the angiographic imaging protocol can be tailored to the requirements of the patient. |
| III | Integrating an analytical model into a NN | According to various examples, it would be possible to determine a contrast-medium enhancement curve for an angiographic imaging protocol based on an estimate of pharmacokinetics of the contrast medium. The estimate of the pharmacokinetics can be obtained from a neuronal network algorithm. This NN can include a known operator layer that implements an analytical model (cf. TAB. 1). |
| | | Accordingly, this option III corresponds to a somewhat inverted scenario of option II. |
| | | In some examples, it would be possible that the analytical model that is integrated into the NN in turn includes at least one free parameter. Then, one or more parameter values of the at least one free parameter can be determined using an optimization algorithm, as already explained in connection with option II above. |

While the analytical models already show robust performance, a data-driven refinement of those analytical models using the NN as exemplified in TAB. 2 can further improve the estimation performance, especially in case of pathologies. NNs, in turn, benefit from the combination with analytical models, as they enable reliable predictions with less data than conventional NNs. Their integration also increases the general reliability of a system with data-driven components, as implausible predictions or learning spurious correlations become unlikely due to a semantic regularization by the analytical models.

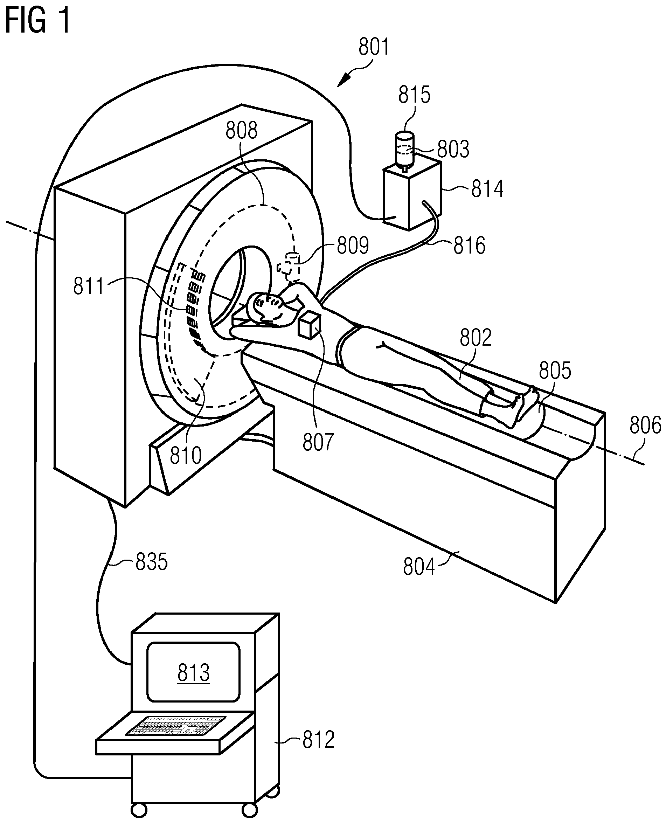

FIG. 1 shows a medical imaging system (diagnostic device), in this example, a CT scanner 801, which can be used to examine a patient 802 using a contrast medium 803 an angiographic imaging protocol. In place of the above-mentioned computed tomography scanner 801, a magnetic resonance device or ultrasonic tomography scanner can also be used.

A patient support table 804 with a movable table panel 805 on which the patient 802 can be supported is associated with the computed tomography scanner 801. The table panel 805 is displaceable in the direction of a rotation axis 806 such that an examination region 807 associated with the patient 802 can be moved through an opening of a gantry 808 of the computed tomography scanner 801 into the measuring region of a recording system 809, 810. The patient 802 and the recording system 809, 810 are displaceable in this way relative to one another in the direction of the rotation axis 806 such that different scanning positions can be assumed. The examination region 807 can be, for example, the heart of the patient. The field-of-view of the measurement may cover the examination region 807 and its surrounding.

In order to detect projections, the recording system 809, 810 has an X-ray emitter 809 in the form of an X-ray tube and an X-ray detector 810 arranged opposed thereto, wherein the X-ray detector 810 is configured arc-shaped and comprises a plurality of detector elements 811 arrayed in detector rows. The X-ray emitter 809 generates radiation in the form of a fan-shaped X-ray beam which penetrates the measuring region and then impinges upon the detector elements 811 of the X-ray detector 810. The detector elements 811 generate an attenuation value dependent on the attenuation of the X-ray radiation passing through the measuring region. The conversion of the X-ray radiation into an attenuation value is carried out, for example, by way of a photodiode which is optically coupled to a scintillator or by way of a direct-conversion semiconductor. In this way, the X-ray detector 810 generates a set of attenuation values which is referred to as a projection. Other detector types are possible, e.g., a photon counting detector.

The recording system 809, 810 is rotatably arranged at the gantry 808 such that projections can be detected from different projection directions. Depending on the operating mode that has been set for the computed tomography scanner 801, the scanning is carried out with a fixed or changing projection direction with a simultaneously fixed or changing scanning position. By rotating the gantry 808 while simultaneously and continuously advancing the patient 802 in the direction of the rotation axis 806, for example, projections are detected from a plurality of different projection directions at different positions along the rotation axis 806 or along the patient 802. The projections obtained in this way by the recording system 809, 810, by way of spiral scanning-rotation of the recording system 809, 810 and simultaneous advancing of the table panel 805-are transferred to a computing device 812 of the imaging system, e.g., the CT scanner 801, and are reconstructed into image data which can be output on a display 813. The image data can comprise, for example, one or more sectional views or 3-dimensional views of the examination region 807.

For the examination of vessels or blood-perfused organs, for example a heart, a liver, in order to enhance the visible contrast in relation to the surrounding soft tissues, a contrast medium 803 can be injected into the patient 802 by way of a contrast medium injector 814. The contrast medium 803 is pumped into a vessel of the patient 802 in a manner that is pre-defined by an injection parameter of the angiographic imaging protocol. Provisioning of the contrast medium 803 is typically time-regulated. The contrast medium 803 is obtained from a supply container 815 via a contrast medium tubing 816 in a settable quantity and at a settable flow rate (multiple syringes may be used). Control commands for contrast medium administration and injection protocols can be transferred via an electrical connection between the computing device 812 and the contrast medium injector 814. According to various examples, it is possible to determine the injection parameter of the angiographic imaging protocol (i.e., parameterize an injection sub-protocol) based on an estimation of pharmacokinetics of the patient.

As the contrast medium 803 spreads out in the interior of the body, the contrast medium 803 introduced passes through the blood circulation of the patient 802, i.e., propagates through the cardiovascular system, and only reaches the examination region 807 after a certain time. To be able to determine this previously unknown delay between the start of the injection and the start of the contrast medium accumulation, before the main scan (CTA), a small quantity of contrast medium, known as the "test bolus", can be injected. Another variant is bolus tracking. On reaching the examination region 807, the concentration of the contrast medium 803 of the test bolus in the blood initially increases, passes through a maximum 818 and subsequently falls again. Multiple CT scans are captured to measure the contrast medium concentration as a function of time. This corresponds to a test bolus measurement. The temporal behavior of the concentration corresponds to a contrast-medium enhancement curve C(t). This can be analyzed afterwards.

Based on such reference bolus measurements, suitable operating parameters of the computed tomography scanner 801 are determined (i.e., the angiographic imaging protocol is configured), such that the main scan is carried out at a time point and a speed at which the concentration of the contrast medium 803 in the examination region 807 is as great as possible. The operating parameters comprise, for example, the start time point of the scanning and the scanning speed and/or the pitch value, that is, the ratio between the advancing of the table panel 805 per rotation of the gantry 808 and the slice thickness of the X-ray detector 810.

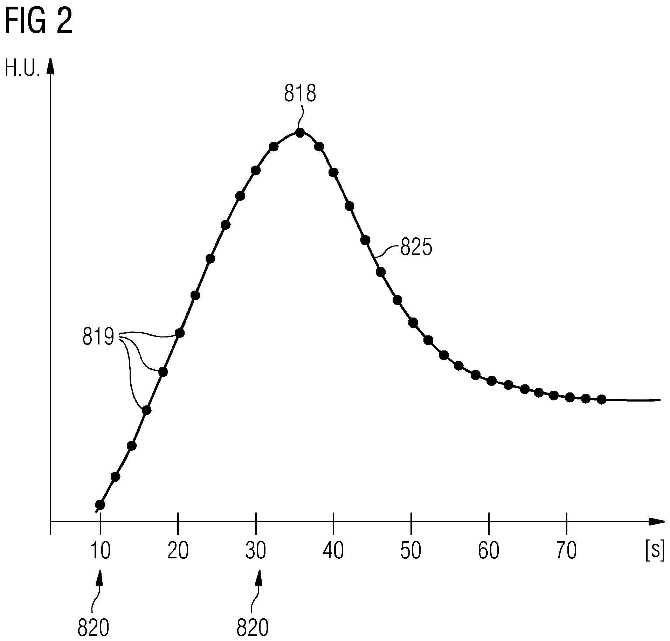

A typical contrast-medium enhancement curve 825 of a test bolus is shown by way of example in FIG. 2. The time after contrast injection t is represented in units of seconds (s) along the x-axis. The y-axis represents the relative attenuation values in Hounsfield units (HU). In the example, attenuation values 819 have been detected every two seconds at scanning time points. This corresponds to regular sampling in time domain; however, according to examples, irregular sampling in time domain would be possible.

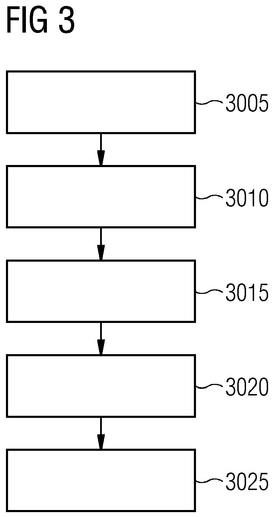

FIG. 3 is a flowchart of a method according to various examples. The method of FIG. 3 can be executed by at least one processor of a computing device, upon loading program code from a memory and upon executing the program code. For instance, the method of FIG. 3 may be executed by the computing device 812 of the CT scanner 801 of FIG. 1.

At optional box 3005, a measurement indicative of a reference contrast medium bolus can be acquired. For this, it would be possible to control the contrast medium injector (cf. FIG. 1: contrast medium injector 814) accordingly. The reference contrast medium bolus measurement can be a test bolus measurement, cf. FIG. 2. Alternatively, a bolus tracking measurement can be executed.

It is generally optional to execute box 3005. Not in all scenarios it is required to use a measurement indicative of the reference contrast medium bolus. For instance, a patient-specific parameterization could also be achieved by considering patient demographics such as age or gender and having respective parameters of an analytical model and/or a NN predetermined for such patient demographics. In some examples, three-dimensional camera images are used as an input to subsequent processing (in addition to or as an alternative to a reference contrast medium bolus).

At box 3010, it is then optionally possible to implement a patient-specific configuration of an analytical model and/or a NN. This may be based on the patient-specific reference contrast medium bolus measurement of box 3005 and/or patient-specific information such as patient demographics. To give an example, an NN can be trained on a physics-informed loss (cf. TAB. 2: option I) which is patient-specific; i.e., an analytical model (cf. TAB. 1) can be used to estimate pharmacokinetics based on a patient-specific reference contrast-medium bolus measurement.

Box 3010 may be executed while the patient is located in the angiographic imaging device, e.g., the CT scanner, e.g., responsive to measuring the test bolus or test bolus tracking. Example patient-specific information that can be considered as part of the patient-specific configuration includes camera data, topograms, information about the heart status, epidemiologic data, specific cardio examinations or anatomic locations. Further examples include age, gender, sex, height, weight physiology e.g. (cardiac output and/or hyper/hypotension and/or vascular resistance and/or family history and/or previous surgery and/or peripheral vascular resistance), lab Values (e.g., ELISA, e(GFR), hematocrit), Medication (e.g., ß blocker, Sublingual nitroglycerin), organ/region of interest , breath hold, organs of interest (e.g. liver, pancreas, spleen, brain, lung, etc.).

For instance, three-dimensional camera images of the patient have been found to provide for a reliable basis to estimate pharmacokinetics of the contrast medium through the patient. Three-dimensional camera images can be obtained, in some examples, using a stereo camera or a time-of-flight camera.

Also contrast and/or scanner specific information may be included, e.g., concentration, temperature, rheology (osmolality/viscosity) and injection parameters such as site, volume, duration, flow-rate, needle size, phases, e.g., uniphase or biphase. Scanner information includes but is not limited to scan speed/duration, tube current, scan direction, tube voltage, (pre)filtration, energy threshold combination.

At box 3015, an estimate of pharmacokinetics of the contrast medium through the cardiovascular system of the patient is determined, e.g., a (e.g., spatially resolved) contrast-medium enhancement curve (cf. FIG. 2).

This corresponds to inference. Ground-truth data is not available.

For box 3010 and box 3015, one or more techniques as outlined in TAB. 2 may be employed. However, it is not required in all scenarios to collate machine learning with analytical models. For instance, according to examples, a neural network may be used that obtains, as input, a reference contrast medium bolus measurement as well as patient-specific information such as a three-dimensional camera image or multiple three-dimensional camera images of the patient.

At box 3020, the angiographic imaging protocol can be configured based on the pharmacokinetics.

At box 3025, the angiographic imaging protocol can be executed, i.e., the angiographic measurement can be executed.

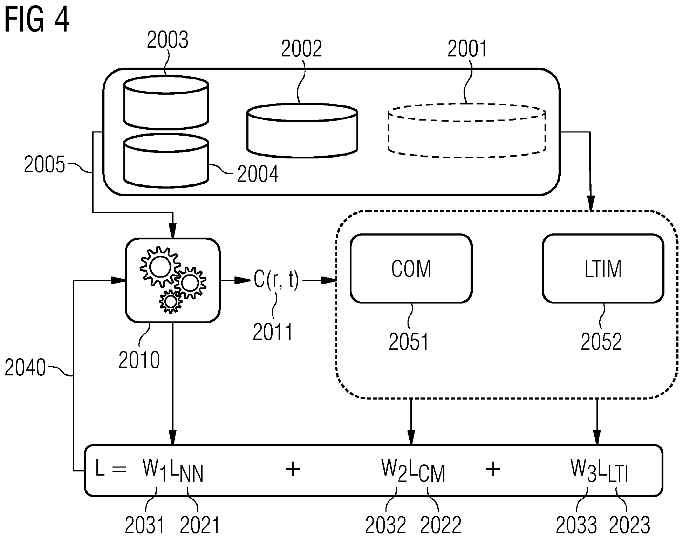

FIG. 4 schematically illustrates a processing pipeline for determining an estimate of pharmacokinetics of a contrast medium through a cardiovascular system of the patient. Specifically, the processing pipeline of FIG. 4 implements the option I of TAB. 2.

A NN 2010 is at the core of the data processing pipeline. The NN 2010 is trained at 2040.

In detail, sampled data points of contrast-medium enhancement curve - test bolus measurement at box 2004 or bolus tracking measurement at box 2003 (cf. FIG. 3: box 3005) - are fed as input data, at 2005, into the NN 2010.

Also, the input can include the time-dependent injection shape of the reference bolus (box 2002).

Optionally, patient-specific information (box 2001), e.g., patient demographics and/or three-dimensional camera image(s), may be input.

Multiple losses 2021, 2022, 2023 can be considered for the training 2040. A first loss 2021, *L_{NN},* is given by the deviation of the estimated contrast-medium enhancement curve C(t) 2010 - which may or may not also be resolved for different positions in the cardiovascular system *C*(*t,r*) - from the measurement of the contrast medium concentration of the reference bolus. A Euclidian distance can be considered. The position argument r can be a ROI (e.g., within the aorta) but also more fine-grained information like the location within the anatomical coordinate system of an organ (e.g., to learn organ perfusion).

At the same time, the estimation of the contrast-medium enhancement curve 2011 provided by the NN 2010 in its current training state is to satisfy constraints imposed by regularization components.

A first helpful constraint is the contrast-medium enhancement curve 2011 should follow the dynamics of a CoM (cf. TAB. 1). A second constraint can be that the contrast-medium enhancement curve is similar to the contrast-medium enhancement curve that is predicted by the system-theoretic LTIM (cf. TAB. 1); again, a Euclidian distance or another distance measure could be minimized. The deviation from the expected dynamics of the CoM is quantitatively captured by the loss ***L_{CM}*** 2022 and the deviation from the LTIM by the loss *L_{LTI}* 2023.

These losses 2022, 2023 work as regularization components in the hybrid model, resulting in more physically meaningful and robust predictions than an approach based solely on (sparse) input data. The losses 2022, 2023 can thus be labelled physics-informed losses. Each of these components can be used separately (indicated by dashed line in FIG. 4).

Further the losses 2021, 2022, 2023 may be weighed with respect to each other. The weights (*w*₁,*w*₂,*w*₃) 2031, 2032, 2033 of the different losses 2021, 2022, 2023 can be determined by, e.g., uncertainty-weighting.

Based on the weighted losses, parameters of the NN 2010 can be adjusted, i.e., the NN 2010 can be trained, 2040. As will be appreciated, this corresponds to a patient-specific training of the NN 2010.

As regards the NN 2010, wavenets may be used. E.g., a recurrent NN or specifically a Long Short Term Memory NN may be used. A transformer NN may be used. However, CNNs are not trivially compatible with irregularly sampled time series data. Therefore, a Neural ODE may be used. Here, the contrast-medium enhancement curve (or more generally pharmacokinetics) is learned by solving the ODE describing the dynamics, which has the benefit of being able to deal with irregularly sampled data, for which also typical systems based on recurrent NN/LSTM or more modern methods like transformers fail. The constraints based on the CoM 2051 and/or the LTIM 2052 as defined above are still imposed, therefore a dynamic is learnt which must not be very distinct from the dynamics described by the CoM 2051 while (optionally) also integrating the information of the LTIM 2052. In further detail: contrast-medium test bolus measurements and also the measured contrast-medium enhancement curve for the diagnostic scan are typical examples of missing data as one only observes parts of the "true" contrast-medium enhancement curve. Neural ODEs as described in R. T. Chen, Y. Rubanova, J. Bettencourt, and D. Duvenaud, "Neural ordinary differential equations," arXiv preprint arXiv:1806.07366, 2018 deal with this kind of data. With this approach not the curve for a new time step is modelled but how the curve changes between time steps, i.e., the dynamics of the change. Conventional modelling with NNs tries to identify a function f(x) that learns y given input data x. Neural ODEs however try to learn ∂y/∂t = f(x, y). This is the formulation of an ODE. Therefore, the optimization pipeline also includes an ODE solver. which results that the loss has to be backpropagated through this solver. The adjoint sensitivity method, see L. S. Pontryagin, Mathematical theory of optimal processes. CRC press, 1987, may be used as an ODE solving method and therefore define adjoint states a(t) that give the sensitivity of the loss with respect to the state. After some steps they arrive at an augmented state that is described by another ODE. The solution of this ODE describing the augmented dynamics then gives the gradients needed to get the parameter update for the next epoch. Instead of Neural ODEs, other advanced variants can be used , e.g., ODE-RNNs and latent ODEs, see Y. Rubanova, R. T. Chen, and D. Duvenaud, "Latent odes for irregularly-sampled time series," arXiv preprint arXiv:1907.03907, 2019; which have shown to cope even better with irregular distributed timepoints. Further examples include stochastic variants of Neural ODEs.

The hyperparameters of the various blocks of FIG. 4 as well as the stopping criterion for the training 2040 can be determined by the predictive performance on the development data after a train/dev/test data split. This process is known as nested cross-validation. Stopping criteria might be a simple early stopping but also more advanced methods like hyperband pruning. An overview of variants can be found in T. Yu and H. Zhu, "Hyper-parameter optimization: A review of algorithms and applications," arXiv preprint arXiv:2003.05689, 2020.

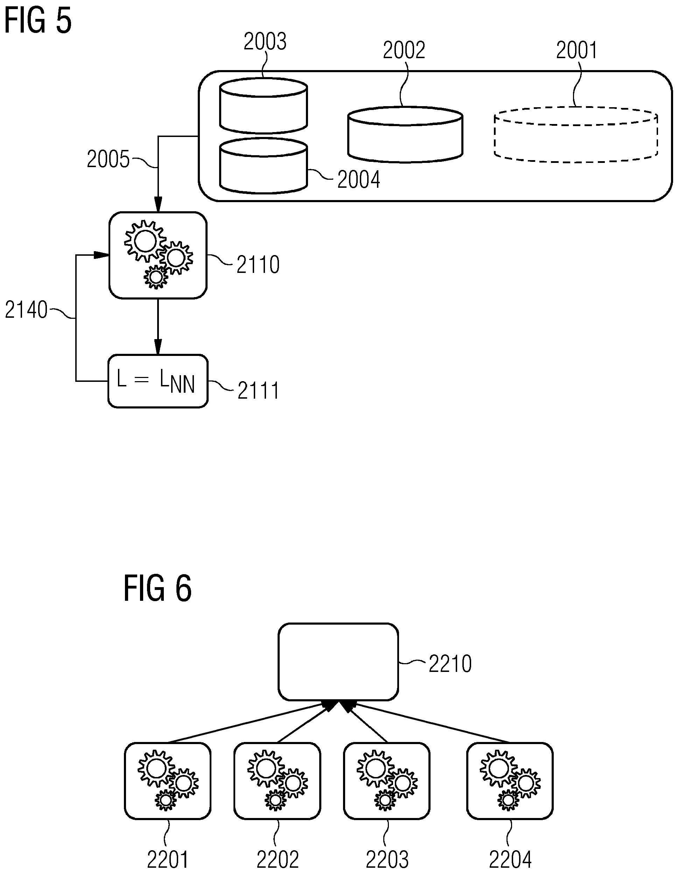

FIG. 5 schematically illustrates a processing pipeline for determining an estimate of pharmacokinetics of a contrast medium through a cardiovascular system of the patient. Specifically, the processing pipeline of FIG. 5 implements the option II and III of TAB. 2.

At the core of the data processing pipeline is a data processing component 2110.

FIG. 5 will be discussed in connection with an implementation of the data processing component 2110 by an LTIM (cf. TAB. 1). Other analytical models may be used, e.g., the CoM, cf. TAB. 1.

The LTIM is based on convoluting a function representing the contrast injection protocol with an analytical function derived by a measurement indicative of a reference contrast-medium measurement bolus.

A first implementation scenario of the data processing pipeline in FIG. 5 (TAB. 2: option II) uses the data processing component 2110 that is based on a modification of the analytical model - e.g., LTIM - by introducing free parameters Θ within the model, either by making fixed parameters in those models learnable or adding additional learnable terms with free parameters which may use, e.g., patient and scan data as arguments (input data 2005 as in FIG. 4). Those free parameters Θ are data-driven components within an otherwise analytical model. Optimization algorithms like genetic algorithms or Bayesian optimization can be used to determine those parameters. Introduced free parameters can also include adjustments to the shape of the function of the contrast injection sub-protocol, which is convoluted with the analytical function derived from test bolus measurement 2004 or bolus tracking measurement 2003.

A second implementation scenario of the data processing pipeline in FIG. (TAB. 2: option III) uses the data processing component 2110 that is implemented by an analytical model e.g., LTIM - that is used as a known operator layer in a NN. Using deterministic models as layers within a NN is termed known operator learning, see A. K. Maier et al., "Learning with known operators reduces maximum error bounds," Nature machine intelligence, vol. 1, no. 8, pp. 373-380, 2019. In this scenario, an analytical component is integrated in a data-driven method.

Both discussed implementation variants (TAB. 2: option II and option III) can be interpreted as a change in degrees of freedom of the respective method, as parameters in a deterministic layer are either made learnable again, therefore increasing the degrees of freedom of the analytical model; or decrease the degrees of freedom of a NN by deterministic, or "analytical", layers.

Also, a combination of both variants is possible. An analytical model with free parameters Θ can be used as a network layer and Θ are learned "on-the-fly" within the optimization of the whole NN - at least if the layer is differentiable.

The training 2140 - e.g., of the free parameter Θ and/or the NN - is illustrated based on a loss 2111 that can correspond to loss 2021 of FIG. 4. Again, a patient-specific training is achieved by considering the analytical model and the loss 2111.

The tuning hyperparameters and stopping criterion can be determined in analogy to what has been explained in connection with FIG. 4.

As will be appreciated from the above, various options of determining the estimate of the pharmacokinetics of the contrast medium through the cardiovascular system of the patient have been disclosed. For instance, in connection with FIG. 4 in FIG. 5, as well as in connection with TAB. 2 various options have been disclosed. According to various examples, it is possible to combine such options. Such techniques are illustrated in connection with FIG. 6.

FIG. 6 schematically illustrates a data processing pipeline for combination of various types of data processing components.

The data processing component 2201 can correspond to a NN that predicts the pharmacokinetics, as explained in connection with TAB. 2: option I. For instance, such estimation can be determined based on a measurement that is indicative of a reference contrast medium bolus, e.g., a test bolus or using bolus tracking. A physics-informed loss can be considered.

The data processing component 2202 can be a CoM. The data processing component 2203 can be an LTIM. The data processing component 2204 can be a NN with extra input information, e.g., a three-dimensional camera image of the patient or body mass index of the patient.

More generally, it is possible to determine multiple pre-estimates of pharmacokinetics of the contrast medium in the cardiovascular system based on multiple models and/or NNs. Then, based on a weighted combination of the multiple pre-estimates, the estimate of the pharmacokinetics can be determined.

These weights can be determined using ensemble learning, at box 2210. The predictions of different models are therefore weighed according to some heuristics. This approach also enables to integrate rare data such as 3-D camera data and to weigh down analytical models for cases in which those models may fail (e.g., due to artifacts). It can also be used when the analytical model (data processing components 2202, 2203) gives implausible results or in case of deficient measured enhancement of a given case (e.g. due to image artifacts). In this artifact case solely the predictions of a data-driven model can be used. Further, this approach enables to integrate data that is only sparsely available due to its novelty and limited availability in the clinical routine (e.g., 3-D camera data; cf. data processing component 2204). If the data is available, the model which uses it as input is used as a part of the ensemble; when the data is not available, the respective input data can be simply omitted.

Further, as a general rule, a lower weighting or non-consideration of predictions based on erroneous analytical methods as disclosed above for ensemble learning can also be solved during the training by a lower weighting or non-consideration of the loss of this analytical component according to option I in TAB. 2, i.e., for this case the analytical component is ignored.

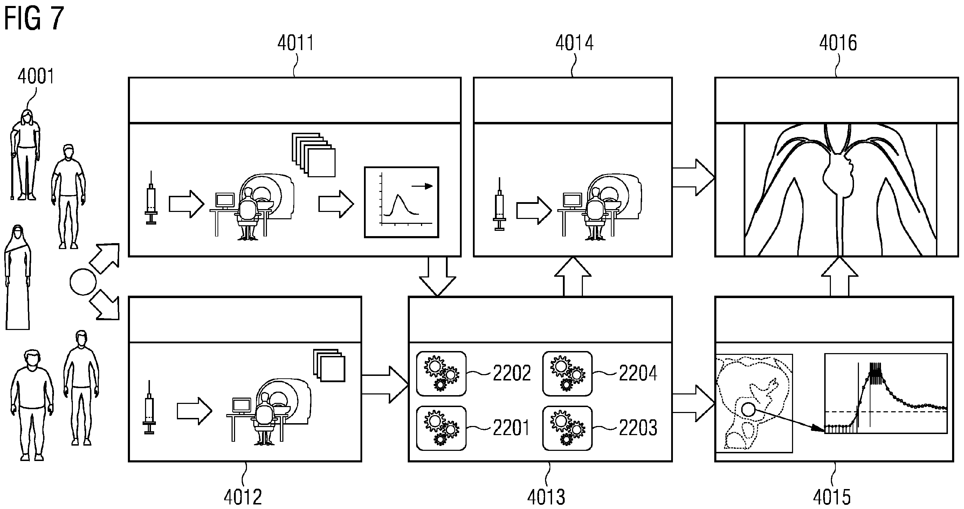

FIG. 7 schematically illustrates a clinical workflow according to various examples.

A patient 4001 is imaged using a medical imaging system for angiography, e.g., the CT scanner 801 as discussed in connection with FIG. 1.

At box 4011, a test bolus workflow can be executed, or at box 4012 a bolus tracking workflow can be executed. I.e., reference contrast-medium bolus measurements can be acquired. This implements box 3005 of the method of FIG. 3.

Based on such input data, one or more data processing components 2201-2204 (cf. FIG. 6) can make (pre-)estimations of the pharmacokinetics of a contrast medium, at box 4013. A scan delay can be calculated. This implements box 3015 of the method of FIG. 3.

It is optionally possible to configure / train these data processing components 2201-2204 in a patient-specific manner (not shown in FIG. 7; corresponding to box 3010 of the method of FIG. 3).

Based on this, and angiographic imaging protocol can be configured (cf. box 3020 of the method of FIG. 3).

Then, at box 4016, diagnostic imaging can be executed using the angiographic imaging protocol. Optionally, it would be possible to validate the configuration of the angiographic imaging protocol using a further test bolus workflow at box 4014 or a further bolus tracking workflow at box 4015. Based on box 4014, 4015 it would also be possible to apply a model or NN that has been configured in a patient-specific manner to a further reference contrast medium bolus measurement to obtain a further revised estimate of the pharmacokinetics.

Summarizing, a hybrid - i.e., analytical and data-driven - data processing has been disclosed to predict patient-specific contrast-medium enhancement curves over time, while refining the analytical method utilizing a function approximator to encode underlying physics laws. This data-driven refinement can be especially useful in cases of pathologies, where the analytical models fail. The data processing is also capable to adapt to the availability of data and provides a high level of safeguarded usage by its ensembled nature and the flexible weighting of its single components (data-driven model, analytical model and extra information).

Clinical implications after successful implementation of the presented optimization method include an improved workflow (increased automation, less user-scanner-interaction, more patient). It also brings in flexibility during protocol design while meeting the requirements for tailored/personalized medicine. Changes in the control UI are not expected (no extra user training necessary).

It is expected that the patient-specific adaptive adjustment of the scan delay leads to increased standardization and better readability, especially for inexperienced customers.

To realize this method no further hardware but solely software updates are required (both, ex-factory scanners and the entire installed will benefit).

It is possible to decrease computational burden to enable real-time application: For example, a NN model based on a PINN can be used for the organs where otherwise physics-based enhancement estimation at inference time of the CoM would require solving heavy ODE layers. This is possible as the PINN has learned with the analytical constraints, such as solving an ODE, and therefore no computation-expensive simulation is needed for inference. This approach has already shown success to approximate simulations, e.g., fluid-simulations, and can be enable real-time application of a computation-expensive analytical method on a scanner.

Ultimately, the optimal and reduced radiation exposure and usage of contrast media leads to cost savings of the respective institution and reduces the risk for the individual patient to experience adverse nephrotoxic side-effects.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For illustrations, techniques have been disclosed that collate the analytical and the NN domain. It is not required in all scenarios to combine one or more analytical models with one or more NNs. For instance, a pure NN may be used that estimates the pharmacokinetics based on one or more camera images, e.g., three-dimensional camera images. The NN may also obtain, as further input, a reference contrast medium bolus measurement.

For further illustration, while above techniques have been disclosed to estimate pharmacokinetics of a contrast medium through a cardiovascular system of a patient, according to various examples, other hemodynamic properties could be estimated, e.g., blood inflow/outflow at certain organs or vessels, etc..

## Claims

1. A computer-implemented method, comprising:
- performing a training of a neural network algorithm (2010, 2201, 2204) to estimate, based on a measurement (2003, 2004) indicative of a reference contrast medium bolus, pharmacokinetics (2011) of a contrast medium (803) through a cardiovascular system of a patient (902, 4001), the training being dependent on at least one loss (2021, 2022, 2023), and
- configuring an angiographic imaging protocol based on an estimate of the pharmacokinetics (2011) of the contrast medium (803) obtained from the neural network algorithm (2010, 2201, 2204),
wherein the at least one loss (2021, 2022, 2023) comprises a loss (2022) determined based on a compartment model (2051, 2202) of the cardiovascular system, the compartment model being based on a fragmentation of the cardiovascular system into multiple compartments and associated differential equations defining a change of a concentration of the contrast medium (803) as a function of time and the concentration of the contrast medium (803) at the respective compartment.

2. The computer-implemented method of claim 1,
wherein the loss (2022) that is determined based on the compartment model (2051, 2202) is based on a comparison between time and spatial derivatives of a prediction of the neural network algorithm (2010, 2201, 2204) for the pharmacokinetics (2011) of the contrast medium (803) and further time and spatial derivatives of the concentration of the contrast medium (803) as defined by the differential equations of the compartment model (2051, 2202).

3. A computer-implemented method, comprising:
- performing a training of a neural network algorithm (2010, 2201, 2204) to estimate based on a measurement (2003, 2004) indicative of a reference contrast medium bolus, pharmacokinetics (2011) of a contrast medium (803) through a cardiovascular system of a patient (902, 4001), the training being dependent on at least one loss, and
- configuring an angiographic imaging protocol based on an estimate of the pharmacokinetics (2011) of the contrast medium (803) obtained from the neural network algorithm (2010, 2201, 2204), and
wherein the at least one loss comprises a loss determined based on a linear time invariant model (2052, 2203) of the cardiovascular system, the linear time invariant model (2052) being based on a response function of a vessel of the cardiovascular system with respect to a time-dependent inflow of the contrast medium (803).

4. The computer-implemented method of any one of the preceding claims,
wherein the measurement comprises time series data that comprises samples that are irregularly spaced in time domain,
wherein the neural network algorithm comprises a Neural Ordinary Differential Equations algorithm.

5. The computer-implemented method of any one of the preceding claims,
wherein the at least one loss (2021, 2022, 2023) comprises multiple losses (2021, 2022, 2023),
wherein the method further comprises:
- weighting (2031, 2032, 2033) the multiple losses.

6. A computer-implemented method, comprising:
- obtaining an estimate of pharmacokinetics (2011) of a contrast medium (803) through a cardiovascular system of a patient (902, 4001), the estimate being obtained from a neural network algorithm (2010, 2201, 2204) that obtains, as input, at least a three-dimensional image of the patient, and
- configuring an angiographic imaging protocol based on the estimate of the pharmacokinetics (2011) of the contrast medium (803).

7. A computer-implemented method, comprising:
- determining one or more parameter values of at least one free parameter of an analytical model (2110) that estimates pharmacokinetics (2011) of a contrast medium (803) through a cardiovascular system of a patient (902, 4001) using an optimization algorithm, the optimization algorithm operating based on an input (2005) comprising a measurement (2003, 2004) indicative of a reference contrast medium bolus, and
- configuring an angiographic imaging protocol based on an estimate of the pharmacokinetics (2011) of the contrast medium (803) obtained from the analytical model (2110).

8. The computer-implemented method of claim 7,
wherein the at least one free parameter comprises a time-dependent in-flow concentration of a contrast-medium measurement bolus.

9. A computer-implemented method, comprising:
- obtaining an estimate of pharmacokinetics (2011) of a contrast medium (803) through a cardiovascular system of a patient (902, 4001), the estimate being obtained from a neural network algorithm (2110) that comprises a known operator layer implementing an analytical model that also estimates the pharmacokinetics (2011) of the contrast medium (803) through the cardiovascular system of the patient (902, 4001), and
- configuring an angiographic imaging protocol based on the estimate of the pharmacokinetics (2011) of the contrast medium (803) obtained from the neural network algorithm.

10. The computer-implemented method of claim 9,
wherein the analytical model comprises at least one free parameter,
wherein the method further comprises:
- determining one or more parameter values of the at least one free parameter using an optimization algorithm, the optimization algorithm operating based on an input comprising a measurement indicative of a reference contrast medium bolus.

11. A computer-implemented method, comprising:
- determining multiple pre-estimates of pharmacokinetics (2011) of a contrast medium (803) through a cardiovascular system of a patient (902, 4001) based on multiple models (2202, 2203) and/or neural network algorithms (2201, 2204),
- based on a weighted combination of the multiple pre-estimates: determining an estimate of the pharmacokinetics (2011), and
- configuring an angiographic imaging protocol based on the estimate of the pharmacokinetics (2011).

12. The computer-implemented method of claim 11, further comprising:
- determining weights of the weighted combination based on ensemble learning.

13. A method executed by at least one processor of a computing device (812) upon loading program code from a memory of the computing device, the method comprising:
- determining one or more estimates of pharmacokinetics (2011) of a contrast medium (803) through a cardiovascular system of a patient (802) based on one or more analytical models and one or more neural network algorithms, and
- configuring an angiographic imaging protocol based on the one or more estimates of pharmacokinetics (2011) of the contrast medium (803).

14. A computing device comprising at least one processor and a memory, the at least one processor being configured to load program code from the memory and to execute the program code, wherein the at least one processor, upon executing the program code, is configured to perform the method of any one of claims 1 to 13.

15. A computer program comprising program code that can be loaded and executed by at least one processor wherein, the at least one processor, upon executing the program code, is configured to perform the method of any one of claims 1 to 13.
